# EUROPÄISCHE PATENTSCHRIFT

(11) **EP 1 940 327 B1**
(45) Veröffentlichungstag und Bekanntmachung des Hinweises auf die Patenterteilung: **06.01.2016**
(21) Anmeldenummer: 06818034.8
(22) Anmeldetag: 25.10.2006
(51) Int. Cl.: A61F 2/76

(54) **Verfahren zur Überprüfung der Einstellung und Einstellung einer Beinprothese sowie Vorrichtung zur Messung von Kräften oder Momenten in einer Beinprothese**
Method for verifying the adjustment and adjusting a leg prosthesis, and apparatus for the measurement of forces or moments in a leg prosthesis
Procédé de surveillance du réglage et réglage d'une prothèse de membre inferieur, ainsi que procédé de mesure de forces ou de couples dans une prothèse de membre inferieur

(30) Priorität: 26.10.2005 DE 102005051646
(43) Veröffentlichungstag der Anmeldung: 09.07.2008
(73) Patentinhaber: Otto Bock Healthcare GmbH, 37115 Duderstadt (DE)
(72) Erfinder: AUBERGER, Roland, A-1020 Wein (AT); PUSCH, Martin, 37115 Duderstadt (DE)
(74) Vertreter: Stornebel, Kai
(86) Internationale Anmeldenummer: PCT/DE2006/001909
(87) Internationale Veröffentlichungsnummer: WO 2007/048404

(56) Entgegenhaltungen:
- EP-A- 1 285 640
- EP-A1- 1 559 384
- WO-A-96/00540
- WO-A2-2005/110293
- GB-A- 1 208 421
- BOENICK U: "Möglichkeiten zur Optimierung der prothetischen Versorgung Beinamputierter mittels quantitativer Bewegungsanalyse" BIOMEDIZINISCHE TECHNIK, Bd. 38, Nr. 6, 1. Juni 1993 (1993-06-01), Seiten 144-152, XP000369834 SCHIELE UND SCHOEN GMBH BERLIN, DE ISSN: 0013-5585

## Beschreibung

Die Erfindung betrifft ein Verfahren zur Überprüfung der Einstellung und zur Einstellung einer Beinprothese, die ein Prothesenkniegelenk aufweist, das einen obenseitigen Anschluss, insbesondere einen Oberschenkelschaft, zur Festlegung der Beinprothese an einem Prothesenträger und einen Unterschenkelschaft, an dem ein Prothesenfuß befestigbar ist, schwenkbar miteinander verbindet, wobei der obenseitige Anschluss oder der Oberschenkelschaft relativ zu einer Gelenkachse verlagerbar ist. Die Erfindung betrifft ebenfalls eine Vorrichtung zur Ermittlung von Kräften und Momenten in einer Beinprothese, z.B. einer Hüftexartikulations- oder Oberschenkelprothese.

Die relative Position zwischen einem Oberschenkelschaft und dem eigentlichen Prothesenkniegelenk ist von einer zentralen Bedeutung für die Funktionalität und den Komfort einer Prothesenversorgung. Diese relative Position hat einen starken Einfluss auf die Sicherheit gegenüber einem unbeabsichtigten Einknicken der Prothese in der Standphase sowie auf die Reaktionskräfte zwischen dem Oberschenkelschaft und dem Oberschenkelstumpf. Besonders wichtig ist dabei die Ausrichtung der Prothese in der Sagittalebene, was auch als "Aufbau" beschrieben wird.

Bei jedem Prothesenaufbau muss ein Kompromiss zwischen einer ausreichenden Sicherheit in der Standphase und einem möglichst geringen Kraftaufwand beim Gehen gefunden werden. Je größer die Sicherheit in der Standphase ist, desto größer ist der Kraftaufwand beim Gehen. Wird der Kraftaufwand beim Gehen minimiert, kann es zu einem instabilen Aufbau des Prothesenkniegelenkes kommen, was durch aktiven Einsatz der Hüftmuskulatur ausgeglichen werden muss. Dies ist für den Prothesennutzer nachteilig.

Die Sicherheit in der Standphase wird grundsätzlich durch eine Rückverlagerung des Prothesenkniegelenkes oder der Gelenkachse relativ zum Oberschenkelschaft erreicht. Dadurch wird garantiert, dass der vom Körper entspringende Gewichtsvektor des Prothesenträgers beim Stehen vor der Gelenkachse des Prothesenkniegelenkes verläuft, wodurch die Prothese in der gestreckten Position verbleibt.

Während eines Schrittes soll die Prothese in die Schwungphase einknicken, dazu muss der Gewichtskraftvektor hinter der Gelenkachse des Prothesenkniegelenkes verlaufen. Dies wird vom Patienten durch Einbringen eines Hüftmomentes erreicht. Die hierzu aufzubringende Körperkraft hängt, wie oben ausgeführt, stark vom Aufbau des Prothesenkniegelenkes ab. Ist der Aufbau zu sicher, so ist das Auslösen in der Schwungphase mit einem großen Kraftaufwand verbunden. Dies führt zu einer vorzeitigen Ermüdung oder auch zu Schmerzen im Oberschenkelstumpf.

Gegenwärtig erfolgt die Positionierung des Prothesenkniegelenkes relativ zu dem Oberschenkelschaft statisch und aufgrund von Erfahrungswerten. Dynamische Effekte, zum Beispiel die Verformung des Systems oder des Oberschenkels während des Gehens, werden nicht berücksichtigt.

Die DE 101 39 333 A1 beschreibt eine Sensoreinrichtung sowie eine Prothese mit einer Sensoreinrichtung, bei der die Sensoreinrichtung in einem Schienbeinteil unterhalb eines künstlichen Kniegelenkes angeordnet ist. Die Sensoreinrichtung sieht einen ringförmig geschlossen ausgebildeten Außenkörper und zwei gegenüberliegende Innenseiten des Außenkörpers verbindende Innenkörper mit einem Sensorelement zur Messung der in Richtung der Verbindungsachse wirkenden Kraft vor. Durch die Sensoreinrichtung können Bodenreaktionskräfte für eine Ganganalyse ermittelt werden.

Die WO 96/00540 A1 betrifft eine Vorrichtung zum Einstellen von Parametern einer Prothese für untere Extremitäten. Eine Vorrichtung ist vorgesehen, mit der eine prothetische Einrichtung in einer horizontalen Ebene in zwei Richtungen senkrecht zueinander verstellt werden kann. Eine vertikale Verstellung über einen Antrieb vorgesehen. Der Antrieb ist mit einem Verlagerungssensor gekoppelt und mit einer Plattform verbunden, die zwei starr miteinander gekoppelte Platten vorsieht. Zwischen den beiden Platten sind Belastungssensoren angeordnet. Ebenfalls sind unabhängige Winkeleinstellmechanismen vorgesehen, um Einstellungen in senkrecht zueinander orientierten Richtungen und zu der Horizontalebene vorzunehmen.

Die EP 1 559 384 A1 betrifft einen Drehmomentsensor für eine Prothese, mit dem eine Messung an einem vorgegebenen Ort eines Bauelementes außerhalb des Messortes möglich ist. Der Drehmomentsensor ist als eine Sensorstruktur aufgebaut, die eine virtuelle Drehachse außerhalb der Sensorstruktur bildet. Messwertaufnehmer sind vorgesehen, die Verformungen der Sensorstruktur zur Bestimmung des Drehmomentes um die virtuelle Drehachse ermöglichen.

Die nachveröffentlichte WO 2005/110293 A2 betrifft ein magnetorheologisch aktuiertes Prothesenknie mit obenseitigen Anschlussmitteln für einen Prothesenschaft, einem Antrieb sowie einem Rahmen, der an einem Gelenkunterteil befestigt ist. An dem Rahmen sind Einrichtungen zur Erfassung von Kräften angeordnet.

Aufgabe der vorliegenden Erfindung ist es, Verfahren und eine Vorrichtung bereitzustellen, mit denen ein Prothesenaufbau eine Oberschenkelprothese verbessert und insbesondere ein günstiger Prothesenaufbau für die Standphase des Gehens ermittelt und eingestellt werden kann.

Erfindungsgemäß wird diese Aufgabe durch Verfahren mit den Merkmalen der Ansprüche 1 und 17 sowie eine Vorrichtung mit den Merkmalen des Anspruchs 19 gelöst. Vorteilhafte Ausgestaltung und Weiterbildung in der Erfindung sind in den Unteransprüchen ausgeführt.

Das erfindungsgemäße Verfahren sieht vor, dass über Sensoren ein Kniemoment und eine in dem Unterschenkelschaft wirkende Axialkraft bei einer Prothesennutzung gemessen werden. Dies geschieht über Sensoren, die bevorzugt im Prothesenkniegelenk und im Unterschenkelschaft angeordnet sind. Aus dem Kniemoment und aus der Axialkraft werden ein resultierender Kraftvektor und die Entfernung des Kraftvektors von der Gelenk- oder Knieachse normal zur Axialkraft errechnet. Der Kraftvektor kann einen Angriffspunkt an dem oberen Anschluss, insbesondere einem Oberschenkelschaft haben. Der Normalabstand relativ zu der Prothesenlängsachse, die die gleiche Richtung wie die Axialkraft hat, und die Lage oder Orientierung des resultierenden Kraftvektors relativ zu der Gelenkachse wird errechnet und aufgrund des Normalabstandes überprüft, ob und wie weit der resultierende Kraftvektor in der Sagittalebene vor oder hinter der Gelenkachse liegt. Liegt der resultierende Kraftvektor vor der Gelenkachse ist das Kniegelenk stabil, liegt er dahinter, spricht man von einem instabilen Prothesenkniegelenk. Es ist auch vorgesehen, im Laufe eines Schrittzyklusses den am weitesten posterior liegenden Kraftvektor zu ermitteln, um die instabilste Situation, die im Laufe des Schrittzyklusses auftritt, betrachten zu können. So können neben der Unterscheidung zwischen einer stabilen und einer instabilen Situation auch Aussagen über den Grad der Instabilität getroffen werden. Hierbei wird insgesamt angenommen, dass der Kraftvektor parallel zu der Axialkraft verläuft, was in der Prothetik überwiegend der Fall ist. Der obenseitige Anschluss kann über einen an dem Oberschenkelstumpf angeordneten Oberschenkelschaft oder osseointegrierte Adapter erfolgen. Bei einer Hüftexartikulation wird als obenseitiger Anschluss die Verbindung von dem Kniegelenk zu dem Hüftgelenk angesehen.

In einer Weiterbildung ist vorgesehen, dass über einen Knöchelmomentsensor das Knöchelmoment zwischen einem Prothesenfuß und dem Unterschenkelschaft erfasst und dass unter Einbeziehung des Knöchelmomentes und der Unterschenkelschaftlänge der resultierende Kraftvektor bestimmt wird. Dadurch ist es möglich, eine Horizontalkomponente einer Bodenreaktionskraft in die Berechnung der Lage des resultierenden Kraftvektors einfließen zu lassen. Der Kraftangriffspunkt des resultierenden Kraftvektors an dem Fuß kann dabei aus dem Quotienten des Knöchelmomentes und der Axialkraft im Unterschenkelschaft errechnet werden. Dadurch wird es möglich, auch bei nicht paralleler Ausrichtung der Axialkraft zu dem Kraftvektor die Lage des Kraftvektors zu ermitteln, wodurch sich eine erhöhte Präzision bei der Berechnung erzielen lässt.

Um die Berücksichtigung der dynamischen Effekte beim Benutzen einer Oberschenkelprothese bei der Ermittlung und Optimierung des Aufbaues zu ermöglichen, ist vorgesehen, das Kniemoment und die Axialkraft, gegebenenfalls auch das Knöchelmoment, während des Gehens zu messen und aufgrund einer Kraft- und Momentenanalyse über die Schrittlänge, im Sinne von Schrittdauer, das Kniemoment bzw. den resultierenden Kraftvektor zu ermitteln, so dass zu jedem Zeitpunkt während des Gehens eine Aussage darüber getroffen werden kann, ob der Aufbau stabil oder instabil ist. So ist es möglich, die Stabilität des Aufbaues der Prothese in der Standphase während des Gehens zu bestimmen, um dadurch dynamische Effekte bei der Evaluierung des Prothesenaufbaues mitberücksichtigen zu können. Aus den Sensordaten kann eine Axialkraft, ein Kniemoment und ein Knöchelmoment ermittelt werden, woraus wiederum der Vektor der Bodenreaktionskraft in der Sagittalebene ermittelt wird. Für eine bessere Visualisierung der Ergebnisse in einem Inertialkoordinatensystem und eine genaue Zuordnung der jeweiligen Werte zu den Phasen eines Schrittes ist es vorgesehen, dass der resultierende Kraftvektor unter Ermittlung des aktuellen Kniewinkels ermittelt wird, beispielsweise über einen Kniewinkelsensor oder einen Oberschenkelwinkelsensor, die die Absolutwinkel relativ zu einer Lotlinie ermitteln. Bei einem bekannten Oberschenkelwinkel kann eine Umwandlung von einem prothesenfesten Koordinatensystem in ein Inertialkoordinatensystem erfolgen. Für die Aufbauanalyse genügt jedoch die Betrachtung eines prothesenfesten Koordinatensystems, die ohne Berücksichtigung des aktuellen Kniewinkels durchgeführt werden kann, wobei eine Identifizierung der Schwungphase über die Axialkraftermittlung durchgeführt werden kann. Ist der Axialkraftwert Null oder werden Zug- statt Druckkräfte gemessen, ist anzunehmen, dass eine Schwungphase vorliegt. Der Kniewinkel kann insbesondere für die Abschätzung des Hüftmomentes eingesetzt werden.

Weiterhin ist es vorgesehen, dass der Winkel zwischen dem Unterschenkelschaft und dem Prothesenfuß ermittelt wird, um eine Plantarflexion des Fußes im Verhältnis zum Unterschenkelschaft zu erfassen. Dies ist erforderlich, da die Spitzfußeinstellung einen starken Einfluss auf die Stabilität oder Instabilität des Prothesenkniegelenkes hat. Durch die Erhöhung des Spitzfußwinkels wird ein überstreckendes und dadurch stabilisierend wirkendes Moment innerhalb des Prothesenkniegelenkes erzeugt, so dass durch eine Änderung des Spitzfußwinkels eine schlechte Rückverlagerungseinstellung teilweise kompensiert werden kann. Insbesondere hat die Spitzfußeinstellung einen großen Einfluss auf den Verlauf des Knie- und des Knöchelmomentes, wobei bei kleiner werdendem Spitzfußwinkel das zum Auslösen der Schwungphase nötige Extensionselement kleiner wird und später mit dem Aufbau des Extensionsmomentes begonnen wird. Je größer der Spitzfußwinkel ist, desto kürzer wird die Ferse des Prothesenfußes belastet. Sollte festgestellt werden, dass der resultierende Kraftvektor in der Standphase beim Gehen hinter der Gelenkachse des Prothesenkniegelenkes angesiedelt ist, also dass es sich in der Standphase um einen instabilen Aufbau handelt, ist vorgesehen, dass die Gelenkachse verstellt bzw. verschoben wird, bis der resultierende Kraftvektor vor der Gelenkachse verläuft oder bis die Gelenkachse hinter dem resultierenden Kraftvektor angeordnet ist. Neben der Kniegelenksachse kann auch eine Gelenkachse des Prothesenfußes verschoben werden, um einen stabilen Prothesenaufbau zu erreichen. Ebenfalls können ergänzend oder alternativ der Streckanschlag des Kniegelenkes und der Streckanschlag des Fußgelenkes, also der Dorsalanschlag, verändert, insbesondere synchron verändert werden. Werden der Dorsalanschlag des Fußgelenkes und der Streckanschlag des Kniegelenkes gegensinnig zueinander verstellt, wird die Flexion des obenseitigen Anschlusses oder Oberschenkelschaftes verändert, was im Vergleich mit einer Verschiebung der Drehachsen nicht zu einander entsprechenden Ergebnissen führt. Die Flexion des Oberschenkelschaftes wäre für den statischen Aufbau nicht ideal, kann jedoch zu Unterscheidung zwischen Stehen und Gehen herangezogen werden. Es wird zur Bestimmung des Aufbaus also ein iterativer Evaluierungsprozess durchgeführt, bei dem die Lage des resultierenden Kraftvektors relativ zu der Gelenkachse bestimmt und der Prothesenaufbau solange verändert wird, bis ein hinreichend stabiler Aufbau in der Standphase im Verlauf des Gangzyklusses erreicht ist. Wünscht der Patient einen dynamischen Aufbau, wird die Prothese so eingestellt, dass der Kraftvektor unmittelbar vor der Gelenkachse verläuft. Das Verfahren und das Aufbaueinstellsystem zeigen jedoch, wo der Kraftvektor verläuft, so dass eine qualifizierte und quantitativ erfasste, ggf. dokumentierte Einstellung des Prothesenkniegelenkes vorgenommen werden kann.

Insbesondere bei elektronisch gesteuerten Knie- oder Fußgelenken kann die Verschiebung der Gelenkachsen beziehungsweise die Veränderung der Streck- oder Dorsalanschläge wirkungsvoll eingesetzt werden, indem zwischen Gehen und Stehen unterschieden wird. Während beim Gehen eine möglichst kleine Rückverlagerung angestrebt wird, um den Kraftaufwand zu minimieren, ist beim Stehen eine sichere Rückverlagerung angestrebt, um einen stabilen Prothesenaufbau und ein sicheres Stehen zu gewährleisten. Über Knöchelmoment- und Kniemomentsensoren werden die aktuell wirksamen Momente ermittelt. Einstellbare Anschläge, beispielsweise sperrbare Hydraulikzylinder mit entsprechenden Aktuatoren stellen in Abhängigkeit von den gemessenen Momenten und der ermittelten Vektorlage die optimale Stellung der Gelenkachsen oder der Anschläge ein, wobei die einzunehmende Stellung anhand empirischer Daten oder persönlicher Vorlieben des Prothesennutzers vorgegeben wird.

Da sich durch die Veränderung des Aufbaues, insbesondere durch die Verschiebung der Gelenkachse oder der Gelenkachsen oder der Anschläge, die Ausrichtung des Prothesenfußes verändert, ist es vorgesehen, vor der Verschiebung der Gelenkachse die relative Position des Prothesenfußes zum Unterschenkelschaft zu markieren und nach der Verschiebung der Gelenkachse die relative Position des Prothesenfußes zum Unterschenkelschaft wieder herzustellen. Dadurch wird gewährleistet, dass die sich durch die Verstellung der Gelenkachse geänderten geometrischen Verhältnisse sich nicht auf die Fußausrichtung auswirken.

Ein einfaches Verfahren zur Markierung und Wiederherstellung der ursprünglichen Prothesenfußeinstellung sieht vor, dass die Relativposition des Prothesenfußes zum dem Unterschenkelschaft ermittelt wird, indem ein Laserpointer fest an dem Oberschenkelschaft angeordnet ist. Ein Laserstrahl wird auf den Prothesenfuß gerichtet und die Auftreffstelle markiert. Nach der Verlagerung der Gelenkachse wird bei einem unveränderten Laserpointer der Prothesenfuß so verstellt, dass die Markierung wieder zur Deckung mit dem Laserstrahl gebracht wird. Statt eines Laserpointers können auch andere, insbesondere optische Markierungsmittel eingesetzt werden.

Nach der Gelenkachsenverschiebung und Ausrichtung des Prothesenfußes kann auch die Plantarflexionseinstellung des Prothesenfußes korrigiert werden. Dazu ist vorgesehen, dass vor der Gelenkachsenverschiebung von dem Oberschenkelschaft ausgehend eine Referenzlinie bis auf den Prothesenfuß angezeichnet und nach der Verschiebung der Gelenkachse der Prothesenfuß so ausgerichtet wird, dass die Referenzlinie wieder zur Deckung gebracht wird, so dass sichergestellt ist, dass der Prothesenfuß wie ursprünglich auf einer Horizontalebene steht. Sofern die Referenzlinie mit der Achse eines Justierkernes zwischen der Kniegelenksprothese und dem Prothesenfuß, also im Bereich des Knöchels, zusammenfällt, kann ein Fehler in der Schaftvorflexion weitgehend vermieden werden. Die Referenzlinie kann durch eine distale Justierachse des Prothesenkniegelenkes gelegt werden.

Eine Weiterbildung des Verfahrens sieht vor, dass das aktuelle Hüftmoment unter Ermittlung des resultierenden Kraftvektors und der gegebenen Prothesengeometrie ermittelt wird. Im Idealfall sollte das Prothesenbein im Stand bzw. während der Standphase nur geringe Hüftmomente aufweisen. Ist das nicht der Fall, sind die Momente in beiden Hüftgelenken nicht gleich, so dass Horizontalkräfte auftreten.

Das Verfahren zur Einstellung einer Beinprothese, die ein Prothesenkniegelenk aufweist, das einen obenseitigen Anschluss zur Festlegung der Beinprothese an einem Prothesenträger und einen Unterschenkelschaft, an dem ein Prothesenfuß befestigt ist, schwenkbar miteinander verbindet, wobei der obenseitige Anschluss relativ zu einer Gelenkachse des Prothesenkniegelenkes und/oder die Position des Prothesenfußes relativ zu dem Unterschenkelschaft verlagerbar ist, sieht vor dass über Sensoren ein Kniemoment und eine Axialkraft bei einer Prothesennutzung gemessen werden, aus denen ein resultierender Kraftvektor errechnet wird, und dass der Normalabstand relativ zu der Gelenkachse und die Lage des resultierenden Kraftvektors zu der Gelenkachse errechnet und überprüft wird, ob und wie weit der resultierende Kraftvektor in Sagittalebene vor oder hinter der Gelenkachse liegt und eine Verstellung des obenseitigen Anschlusses relativ zu der Prothesenkniegelenkachse und/oder Streckanschläge für das Prothesenkniegelenk und/oder ein Prothesenfußgelenk in Abhängigkeit von der ermittelten Lage des resultierenden Kraftvektors vorgenommen wird.

Die Kniegelenksachse, die Fußgelenksachse und/oder die Streckanschläge können so eingestellt werden, dass der resultierende Kraftvektor während der Standphase vor der Kniegelenksachse liegt.

Die erfindungsgemäße Vorrichtung zur Messung von Kräften oder Momenten in einer Beinprothese, insbesondere in einer Oberschenkel- oder Hüftexartikulationsprothese, mit einem Prothesenkniegelenk zur Überprüfung der Einstellung oder zur Einstellung eines Prothesenaufbaues sieht vor, dass ein Oberteil zur Verbindung mit einem obenseitigen Anschluss, insbesondere Oberschenkelschaft, und ein mit dem Oberteil gelenkig verbundenes Unterteil vorhanden ist, wobei Anschlussmittel zur Befestigung des Oberteiles an dem obenseitigen Anschluss vorgesehen sind, wobei die Anschlussmittel bevorzugt einen in der Sagittalebene verschieblich an dem Oberteil gelagerten Adapter aufweisen und Einrichtungen zur Erfassung von Kniekräften oder Kniemomenten an den Anschlussmitteln oder an dem Oberteil angebracht sind. Durch die Vorrichtung ist es möglich, die wirksamen Kräfte und Momente innerhalb eines Prothesenkniegelenkes zu erfassen und darüber hinaus bevorzugt durch die Einstellung der Anschlussmittel die Lage der Gelenkachse relativ zu dem Oberschenkelschaft so zu ermitteln oder zu verändern, dass ein optimaler Protheseriaufbau erzielt werden kann. Der Adapter kann dabei an dem Oberteil über eine Klemmeinrichtung festgelegt werden. Alternativ dazu sind von außen zugängliche Einstellschrauben beispielsweise in Gestalt einer Spindel vorgesehen, über die bequem die Gelenkachse relativ zu dem Oberteil oder dem obenseitigen Anschluss, insbesondere einem Oberschenkelschaft verschoben, damit der Prothesenaufbau verändert und die somit gewonnenen Erkenntnisse über den Prothesenaufbau unmittelbar in der Praxis überprüft werden können.

Eine Ausgestaltung der Erfindung sieht vor, dass zwischen dem Oberteil und dem Adapter ein Brückenelement und/oder ein Träger angeordnet sind, auf der der Adapter verschieblich gelagert ist. Die Brücke oder der Träger ist dabei bevorzugt momentenstarr an dem Oberteil festgelegt und bildet zwischen dem unmittelbar dem Oberschenkelschaft angeordneten Adapter und dem Oberteil ein Zwischenstück, das zur Ermittlung der in dem Kniegelenk wirksamen Kräfte und Momente entsprechend ausgestaltet ist. An der Brücke kann ein Messbalken ausgebildet sein, der sich bei Beaufschlagung mit Momenten oder Kräften in einem solchen Maße verformt, dass beispielsweise über Dehnungsmessstreifen die wirksamen Kniekräfte und Kniemomente eindeutig mit einem hohen Messsignal ermittelt werden können. Es entsteht so eine preisgünstige Ausgestaltung einer bidirektionalen Messeinheit. Der Messbalken muss jedoch so dimensioniert sein, dass die Brücke nicht versagt und auch bei Erreichen von Werten der Maximalbelastung die Struktur erhalten bleibt. Bevorzugt sind der Adapter bzw. der Adapter mit der Brücke und dem Träger so gestaltet, dass die Einbauhöhe der Einbauhöhe der Anschlussmittel entspricht, die nach der Einstellung des Prothesenaufbaues eingesetzt werden. Bei dieser Variante ist vorgesehen, dass nach Ermittlung des optimalen Prothesenaufbaus die Vorrichtung entfernt und durch die normalen Anschlussmittel ersetzt wird. Nachfolgend wird ein Ausführungsbeispiel der Erfindung anhand der Figuren näher erläutert. Es zeigen:
- Figur 1 -: eine schematische Darstellung einer Beinprothese mit darin angeordneten Sensoren;
- Figur 2 -: eine Darstellung der wirksamen Kräfte in einer Anordnung gemäß Figur 1;
- Figur 3 -: eine perspektivische Darstellung der Momente und Kräfteverhältnisse sowie Winkel;
- Figur 4 -: eine schematische Darstellung der Figur 3;
- Figur 5 -: den Zusammenhang zwischen einem Kniemomentenverlauf und einer Rückverlagerung;
- Figur 5a -: eine schematische Darstellung einer Einstellung von Streckanschlägen;
- Figur 6 -: eine schematische Darstellung der Wiederherstellung der Fußposition;
- Figur 7 -: eine schematische Darstellung der Wiederherstellung der Plantarflexion;
- Figur 8 -: ein Ausführungsbeispiel der erfindungsgemäßen Vorrichtung im zusammengebauten Zustand;
- Figur 9 -: eine Variante der Figur 8 in gedrehter Darstellung;
- Figur 10 -: eine Draufsicht auf die Vorrichtung; sowie
- Figur 11 -: eine Schnittdarstellung entlang A-A der Figur 10.

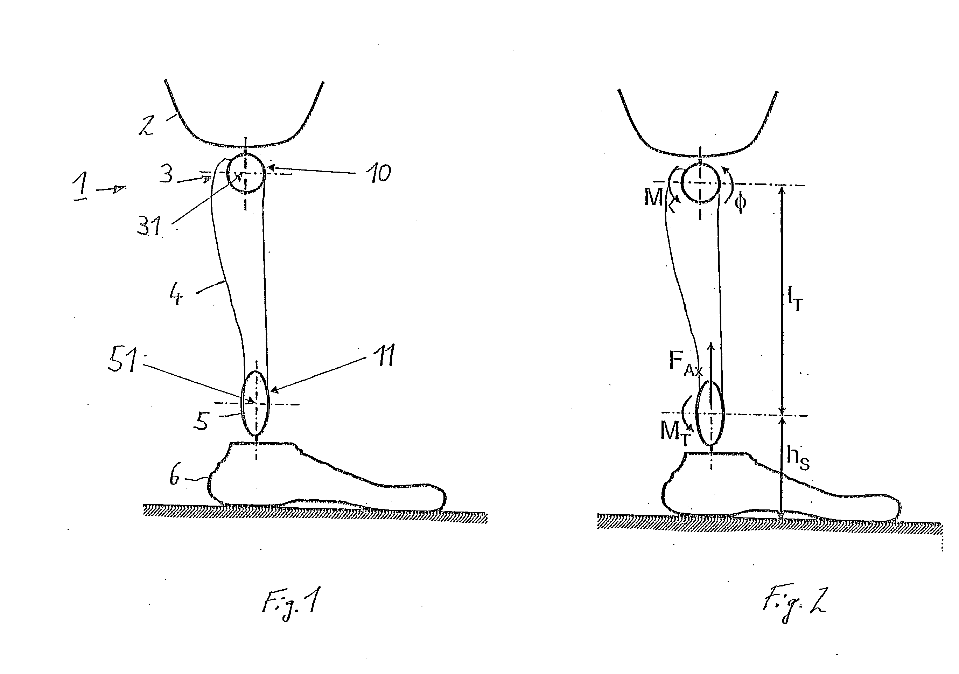

In der Figur 1 ist eine Beinprothese 1 mit einem Oberschenkelschaft 2, der an einem nicht dargestellten Oberschenkelstumpf befestigbar ist, dargestellt. An dem Oberschenkelschaft 2 ist ein Kniegelenk 3 befestigt, das um eine Knieachse 31 verschwenkbar gelagert ist. An dem Prothesenkniegelenk 3 ist ein Unterschenkelschaft 4 befestigt, der das Kniegelenk 3 mit einem Prothesenfuß 6 über eine Koppelstelle 5 verbindet. In dem Kniegelenk 3 ist ein Sensor 10 zur Messung des Kniemomentes M_{K} angebracht, wobei die Messachse 31 des Sensors 10 der Gelenkachse des Knies 3 entspricht. Der Kniewinkel Φ kann entweder über den Sensor 10 oder einen anderen, beispielsweise in dem Unterschenkelschaft 4 Sensor ermittelt werden. Alternativ zu einem Oberschenkelschaft 2 kann ein osseointegrierter, obenseitiger Anschluss vorgesehen sein, der das Kniegelenk 3 mit dem Oberschenkelstumpf verbindet. Bei Hüftexartikulationen ist kein Oberschenkelstumpf vorhanden, so dass die Verbindung vom Kniegelenk 3 zum Hüftgelenk als obenseitiger Anschluss angesehen wird.

Zur Bestimmung eines Knöchelmomentes M_{T} ist im Anschlussbereich zwischen dem Unterschenkelschaft 4 und dem Prothesenfuß 6 ein Sensor 11 zur Messung einer innerhalb des Unterschenkelschaftes 4 wirksamen Axialkraft F_{AX} und des Knöchelmomentes M_{T} angeordnet, die Messachse 51 des Sensors 11 liegt parallel zu der Knieachse 31.

In der Figur 2 sind die Höhen und Längen sowie die Orientierungen der Kräfte und Momente eingezeichnet, die Höhe des Knöchelmomentsensors 11 bzw. der Sensorachse 51 wird mit H_{S} bezeichnet.

Der Abstand der Knieachse 31 zur Messachse 51 des Knöchelmomentsensors 11 wird mit L_{T.} bezeichnet. Das Knöchelmoment M_{T} ist ebenso wie das Kniemoment M_{K} entgegen dem Uhrzeigersinn positiv eingezeichnet, ebenso wie die Axialkraft F_{AX} im Unterschenkelschaft 4 nach oben wirkend, also in Richtung des Kniegelenkes 3 positiv angenommen wird.

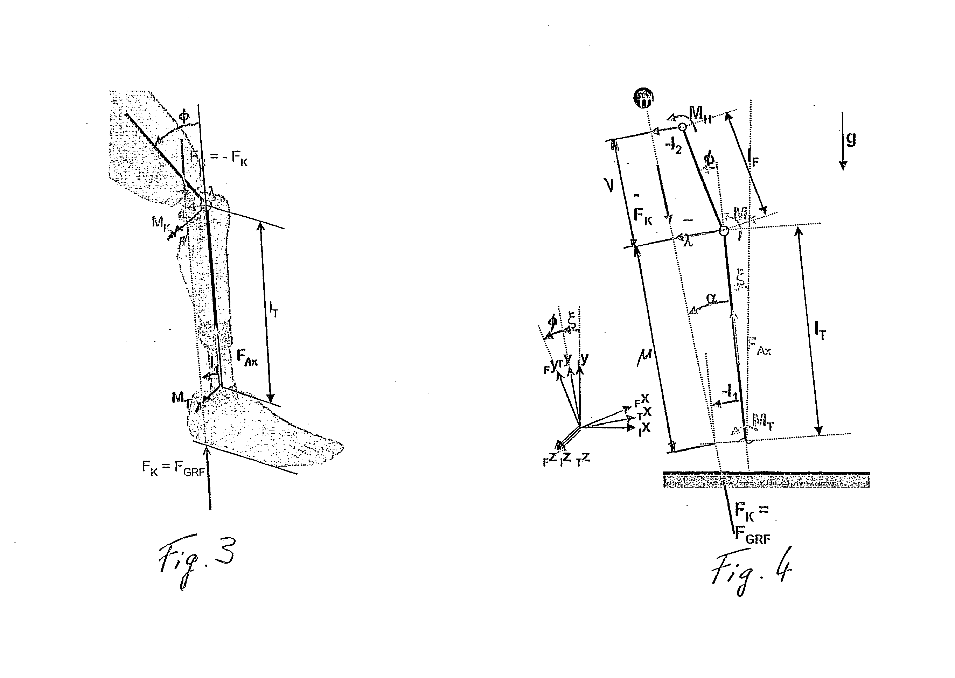

In der Figur 3 sind weitere wirksame Kräfte perspektivischer Darstellung gezeigt, wobei die eine Kontaktkraft F_{K} als die Bodenreaktionskraft F_{GRF} angenommen wird, die immer, gemäß dem Koordinatensystem in der Figur 4, als in y-Richtung positiv wirksam angesehen wird. Ist der Oberschenkelschaft 2 um einen Winkel Φ entgegen dem Uhrzeigersinn verschwenkt, wirkt eine resultierende Kraft F_{R} der Kontaktkraft F_{K} entgegen, wobei sich aus dem Abstand λ als Normalabstand des Kraftvektors F_{R} von der Knieachse 31 ableiten lässt, ob der momentane Aufbau des Prothesenkniegelenkes 3 stabil oder instabil ist. Die Aussagen des Modells gelten für jedes betrachtet Koordinatensystem, also auch für ein unterschenkelfestes Koordinatensystem. Sofern eine Abschätzung der Verhältnisse an der Hüfte nicht erforderlich oder uninteressant ist, werden keine Kniewinkelinformationen benötigt.

In der in der Figur 4 dargestellten Situation eines Fersenauftrittes mit einem Winkel ξ zwischen der Prothesenlängsachse und dem Lot ist λ negativ, so dass der resultierende Kraftvektor F_{R} in Sagittalebene hinter der Gelenkachse 31 liegt. Das Prothesenkniegelenk 3 wäre somit instabil. Um einen stabilen Prothesenaufbau bereitstellen zu können, müsste die Gelenkachse 31 in negative x-Richtung in der Sagittalebene verlagert werden. Eine Rückverlagerung der Gelenkachse 31 hat somit auf die Verläufe von Kniemoment, Knöchelmoment und Axialkraft wesentlichen Einfluss.

Bei einem stabilen Prothesenaufbau sollte das Kniemoment zu einem gegebenen Zeitpunkt in der Standphase aufgrund der vorliegenden Mechanik, also aufgrund der vorliegenden Hebellängen, linear von der Rückverlagerung abhängen. Ist der Prothesenaufbau sehr instabil, muss der Prothesenträger durch Aufbringen eines Hüftstreckmomentes aktiv verhindern, dass die Prothese unter ihm wegknickt. Dies wirkt sich theoretisch als eine Reduktion des maximalen Kniemomentes in der Standphase aus. Grundsätzlich soll eine Prothese so aufgebaut sein, dass eine Standphaseflexion möglich ist, da dadurch ein Kontaktstoß beim Aufsetzen der Prothese auf dem Boden reduziert wird. Aus diesem Grunde sollte der Prothesenaufbau nicht zu weit rückverlagert sein, um ein gewisses Beugemoment zu ermöglichen, jedoch ohne den Prothesenträger dazu zu zwingen, aktiv ein weiteres Einknicken der Prothese zu verhindern. Jedoch ist bei einem Prothesenaufbau auf die individuellen Wünsche des Prothesennutzers einzugehen. Das in der Standphase zulässige Flexionsmoment ist stark individuellen Unterschieden unterworfen, die auch dynamischen Einflüssen unterliegen, so dass die optimale Ausrichtung der Gelenkachse in der Regel nicht in einem Ausrichten in statischem Zustand erfolgen kann, sondern bislang durch mühsame Versuchsreihen ermittelt werden musste. Diese Einstellprozedur kann mit dem erfindungsgemäßen Verfahren und der erfindungsgemäßen Vorrichtung wesentlich verkürzt werden.

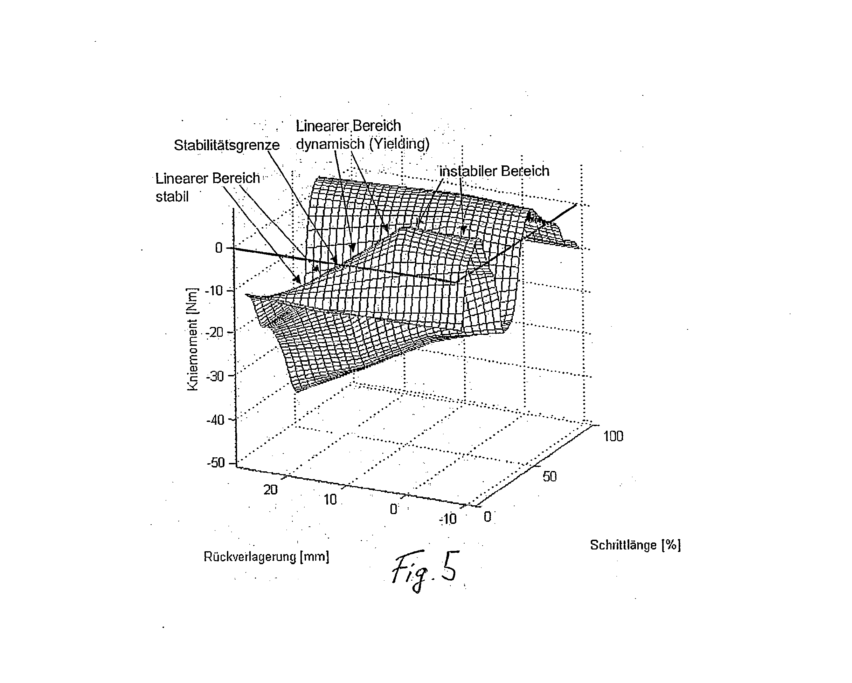

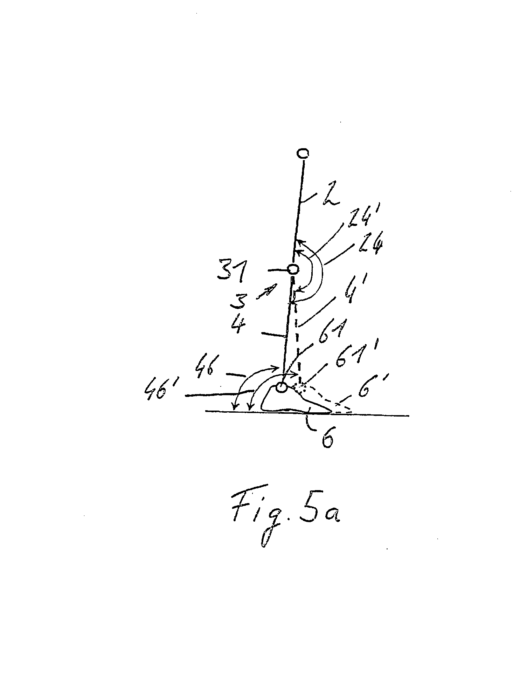

In der Figur 5 ist exemplarisch der Zusammenhang zwischen einem Kniemomentenverlauf und einer Rückverlagerung über einen Schrittzyklus gezeigt. Die Zeit ist hierbei normiert als prozentuale Schrittlänge, wobei 100% einem vollen Schrittzyklus entspricht. Auf der zweiten Horizontalachse ist die Rückverlagerung der Gelenkachse bezüglich einer Montagebezugslinie, also der Verbindungslinie von der Unterschenkelschaftmitte zur Prothesenfußmitte, in Millimetern dargestellt. Bei einem Kniemomentwert von Null liegt der Übergang von einem stabilen zu einem instabilen Prothesenaufbau vor. Bei einem positiven Kniemoment ist ein instabiler Prothesenaufbau erreicht. Bei einem Übergang von einem stabilen zu einem instabilen Aufbau wird eine Stabilitätsgrenze erreicht, die ein sinnvoller Richtwert für die Einstellung des Prothesenaufbaues ab der Standphasenflexion ist. Ist die Lage dieser Stabilitätsgrenze bekannt, kann leicht auf die individuellen Bedürfnisse eines Prothesennutzers eingegangen werden, indem bei einem gewünschten stabilen Aufbau die Prothese rückverlagert wird; bei einem gewünschten dynamischen Aufbau kann eine Vorverlagerung stattfinden.

Ein weiterer Parameter mit einem Einfluss auf die Stabilität des Prothesenaufbaues ist die Spitzfußeinstellung. Durch eine Erhöhung des Spitzfußwinkels mit mehr Plantarflexion wird ein überstreckendes und dadurch stabilisierend wirkendes Moment im Knie erzeugt. Grundsätzlich kann man eine suboptimale Rückverlagerungseinstellung durch Änderung der Spitzfußeinstellung kompensieren, wobei bei einem großen Spitzfußwinkel das Prothesenkniegelenk sehr früh überstreckt wird. Das erforderliche Extensionsmoment zum Auslösen der Schwungphase ist dann groß und muss für eine verhältnismäßig lange Zeit aufgebracht werden. Je kleiner der Spitzfußwinkel ist, desto kleiner ist das zum Auslösen der Schwungphase nötige Extensionsmoment, wobei auch später mit dem Aufbau eines Extensionsmomentes begonnen wird. Auch hat der Spitzfußwinkel einen Einfluss auf den Kniemomentverlauf während des Belastens der Prothese, wobei das Ziel der Einstellung des Spitzfußwinkels darin besteht, einen möglichst gleichmäßigen Kniemomentverlauf bereitzustellen.

Um die Einstellung eines Prothesenkniegelenkes während der Benutzung überprüfen zu können, ist es notwendig, den Normalabstand λ des resultierenden Kraftvektors F_{R} von der Knieachse 31 zu jedem Zeitpunkt bzw. Abtastzeitpunkt während der Standphase zu ermitteln. Um den Normalabstand λ zu erhalten, ist es notwendig, die Orientierung und den Angriffspunkt des resultierenden Kraftvektors F_{R} bzw. der Bodenreaktionskraft F_{K} zu ermitteln. Da für die Beurteilung, ob ein stabiler oder instabiler Zustand des Prothesenaufbaus in der Standphase vorhanden ist, nur eine Verlagerung innerhalb der Sagittalebene zu betrachten ist, liegt ein ebenes Problem vor. Aus dem Knöchelmomentsensor 11 sowie dem Sensor zur Ermittlung der Axialkraft F_{AX} und dem Sensor 10 zur Ermittlung des Kniemomentes M_{K} sowie die vorher ermittelten Längen Hs und L_{T} ist es möglich, den resultierenden Kraftvektor F_{R} oder die korrespondierende Kontaktkraft F_{K} in Lage und Orientierung zu ermitteln. Dabei kann alles, was oberhalb des Kniegelenkes 3 passiert, auf die resultierende Kraft F_{R} reduziert werden, was insbesondere bedeutet, dass keine aktiv aufgebrachten Hüftmomente M_{H} berücksichtig werden und dass der Einfluss eines Horizontalanteiles der Bodenreaktionskraft F_{GRF} auf das Knöchelmoment M_{T} vernachlässigbar ist. Die Annahme, dass das Hüftmoment M_{H} vernachlässigbar ist, kann dann zutreffenderweise getroffen werden, wenn der Prothesenaufbau nicht extrem instabil ist. Um Hüftmomente M_{H} berücksichtigen zu können, müsste zumindest ein weiterer Sensor vorgesehen sein, was zu aufwändig wäre.

Um das Hüftmoment M_{H} abschätzen zu können, kann über das Momentengleichgewicht am Hüftgelenk mit M_{H}= L₂*F_{K} und der Beziehung L₂=L_{F} * sin(Φ- a) + λ eine Berechnung bei ermittelter Kontaktkraft F_{K} erfolgen, mit L_{F} als der gemessenen Oberschenkellänge und L₂ als Abstand des resultierenden Kraftvektors vom Angriffspunkt des Hüftmomentes M_{H} mit dem Kniewinkel Φ. Um die effektive Hebellänge L₁ auf Höhe des Knöchelmomentsensors 5 berechnen zu können, wird der Quotient aus dem gemessenen Knöchelmoment M_{T} und der Axialkraft F_{AX} im Unterschenkelschaft gebildet. Die Hebellänge L₁ gibt die Entfernung des Krafteintrittspunktes vom Knöchelgelenk auf der Höhe des Knöchelmomentsensors 11 an. Das Momentengleichgewicht am Sprunggelenk lautet M_{T} = - L₁ X F_{K}

Zur expliziten Berechnung der Vertikalkomponente der Kontaktkraft F_{K} wird die Gleichung **M_{T} = F_{AX} · L₁ - F_{KX} · H_{S}** entsprechend aufgelöst. Das Kniemoment M_{K} entspricht dem negativen Produkt aus Normalabstand λ und der Kontaktkraft F_{K}. Aus dem Kräftegleichgewicht an dem Unterschenkelschaft 4 mit F_{K} cos(α) + F_{AX} = 0 und der geometrischen Beziehung sin(α) = (λ-L₁)/L_{T} ergibt sich ein nichtlineares Gleichungssystem, aus dem sich die gesuchten Werte errechnen lassen.

Dabei ist der Winkel α der Winkel zwischen dem Kraftvektor und der Prothesenlängsachse. F_{KX} sowie F_{KY} sind Kontaktkraftkomponenten in X- und Y-Richtung. Die Errechnung der benötigten Größen F_{KX}, F_{KY}, L₁ und λ erfolgt in einem Computer, in den die geometrischen Größen und Abmessungen sowie die gemessenen Momente, Winkel und Kräfte eingegeben werden. Die aktuellen Messwerte können über eine Schnittstelle in den Rechner gesendet werden, die Abmessungen können manuell eingegeben werden. Aus der Tatsache, dass der Normalabstand λ des Bodenreaktionsvektors F_{GRF} oder des Kontaktkraftvektors F_{K} von der Knieachse 31 zu jedem Zeitpunkt der Standphase berechnet werden kann, kann auch zu jedem Zeitpunkt der Standphase ermittelt werden, ob ein stabiler oder instabiler Prothesenaufbau vorhanden ist. Ob ein stabiler oder instabiler Aufbau vorliegt, kann aus einem Vergleich des ermittelten Abstandes mit einem definierten Abstand für einen stabilen oder instabilen bzw. dynamischen Aufbau erfolgen.

Um den Prothesenaufbau weiter zu optimieren, muss die Spitzfußeinstellung so gewählt werden, dass der Kraftvektor im Stehen durch die Fußmitte verläuft. Dies kann durch eine Wippenanordnung realisiert werden, auf die sich eine Person mit der Prothese dergestalt stellt, dass sich die Plattenmitte an der gewünschten Lastanleitungsposition des Prothesenfußes befindet. Mittels einer Markierung auf der Platte kann die Fußmitte über den Drehpunkt gebracht werden, wobei Ferse und Zehe gleichweit von der Markierung beabstandet sein müssen. Bei einem entspannten Stehen wird die Plantarflexion solange geändert, bis die Platte im Gleichgewicht ist und auf einem unterhalb der Markierung angebrachten Stab wippt. Sobald das Gleichgewicht erreicht ist, liegt der Kraftursprungspunkt in der Fußmitte.

Nach der durchgeführten Plantarflexionseinstellung und der Messung der Momente und Kräfte innerhalb der Prothese und der Ermittlung des Prothesenaufbaucharakters wird die Gelenkachse um einen Betrag λ' in der Sagittalebene nach vorn oder hinten verschoben. Die notwendige Verschiebung λ' zum Erreichen eines Zielmomentes im M' im Kniegelenk 3 in der Standphasenflexion kann errechnet werden. Alternativ kann die Verschiebung durch eine gegensinnige Veränderung der Streckanschläge um arctan (λ'/L_{T}) erfolgen.

In der Figur 5a ist eine schematische Darstellung einer Beinprothese gezeigt, bei der der Oberschenkelschaft 2 über das Prothesenkniegelenk mit der Gelenkachse 31 mit dem Unterschenkel 4 verbunden ist. Der Unterschenkel 4 trägt an seinem distalen Ende einen Prothesenfuß 6, der über eine Gelenkachse 61 verschwenkbar und gegebenenfalls einstellbar an dem Unterschenkelschaft 4 befestigt ist. Zwischen dem Unterschenkelschaft 4 und dem Oberschenkelschaft 2 befindet sich ein einstellbarer Streckanschlag 24, der durch einen Pfeil angedeutet ist. Dieser Streckanschlag 24 kann verändert werden, beispielsweise dergestalt, dass der Prothesenfuß 6 weiter vorgelagert wird. Dann befindet sich der Prothesenfuß 6 in der gestrichelten Position 6', während der Unterschenkelschaft 4' in Gehrichtung nach vorne geschwenkt ist. Der dann eingenommene Streckanschlag 24' des Kniegelenkes 3 wurde angepasst, um in Abhängigkeit von der ermittelten Position des resultierenden Kraftvektors einen stabilen oder dynamischen Aufbau zu realisieren. Gleichzeitig kann entweder synchron der Dorsalanschlag 46 des Prothesenfußes 6 eingestellt werden. Der dann vorhandene Dorsalanschlag 46' bewirkt eine Anpassung der Fußstellung. Die Verstellung der Streckanschläge 24, 46 kann elektronisch gesteuert über Aktuatoren, beispielsweise verriegelbare Hydraulikzylinder geschehen. Eine Anpassung kann somit an unterschiedliche Prothesennutzer oder unterschiedliche Einsatzbedingungen erfolgen. Ebenfalls ist es möglich, dass bei einer Verschiebung des Unterschenkels 4 relativ zur Gelenkachse 31 die Prothesenfußstellung automatisch korrigiert wird, so dass ein Prothesenaufbau nach den Vorstellungen des Prothesennutzers in Abhängigkeit von dem tatsächlich vorhandenen Prothesenaufbau, den Einsatzbedingungen und der Position der Prothese an dem Prothesennutzer realisiert werden kann.

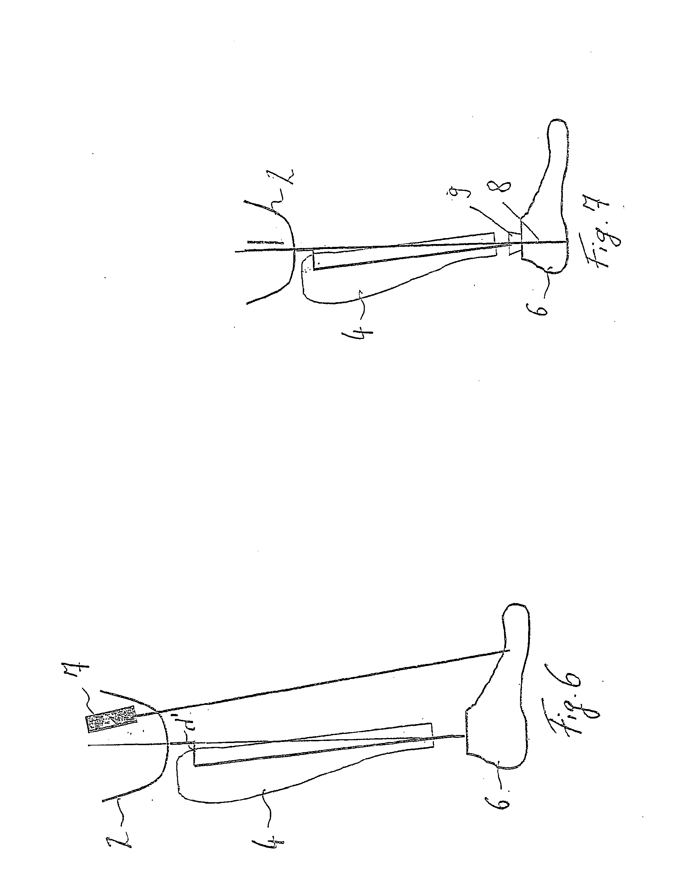

Um nach einer Anpassung der Rückverlagerung durch Verstellung der Knieachse 3 die Fußposition wie gehabt auszurichten, wird wie in der Figur 6 gezeigt, vor der Verstellung über einen an dem Oberschenkelschaft 2 befestigten Laserpointer 7 ein Lichtfleck auf der Oberseite des Prothesenfußes 6 aufgebracht und markiert. Anschließend wird die Gelenkachse 3 um den errechneten Betrag λ' verschoben. Nach der Verschiebung wird die Prothese solange gedreht, bis der projizierte Punkt wieder mit der Markierung zusammenfällt.

Ebenfalls sollte vor der Rückverlagerung die Spitzfußeinstellung bzw. Plantarflexion des Prothesenfußes 6 kontrolliert werden. Dies erfolgt vorteilhafterweise nach der Ausrichtung des Prothesenfußes wie oben beschrieben über einen Laserpointer. Bei der Wiederherstellung der ursprünglichen Plantarflexion werden Schrauben an einer Knöchelpyramide 9 gelockert, der Prothesenträger steht bequem, damit sich das System setzen kann und die Schuhsohle satt auf dem Boden aufliegt. Dann werden die Schrauben wieder angezogen. Alternativ kann zur Einstellung der Plantarflexion auch die oben beschriebene Wippe verwendet oder vor der Rückverlagerung eine durchgehende Referenzlinie 9 an dem Schaft 4 und dem Prothesenfuß 6 angezeichnet werden. Nach der Rückverlagerung werden die ggf. zueinander versetzten Linienteile zueinander fluchtend ausgerichtet, so dass die ursprüngliche Spitzfußeinstellung wiederhergestellt ist. Nach der ersten Rückverlagerung, der Ausrichtung des Prothesenfußes 6 sowie der Plantarflexionskorrektur wird der Prothesenaufbau erneut über mehrere Schrittzyklen auf einer ebenen Fläche evalüiert und gegebenenfalls korrigiert.

Abschließend werden die auftretenden Maximalbelastungen kontrolliert. Sofern eine zufriedenstellende Einstellung gefunden wurde, kann der Momentensensor im Prothesenkniegelenk 3 durch einen passend eingestellten Verschiebeadapter ersetzt werden.

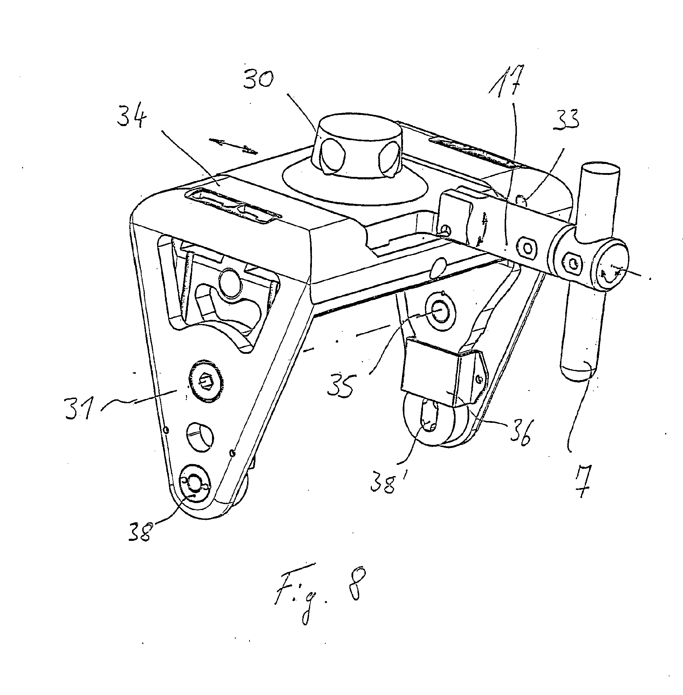

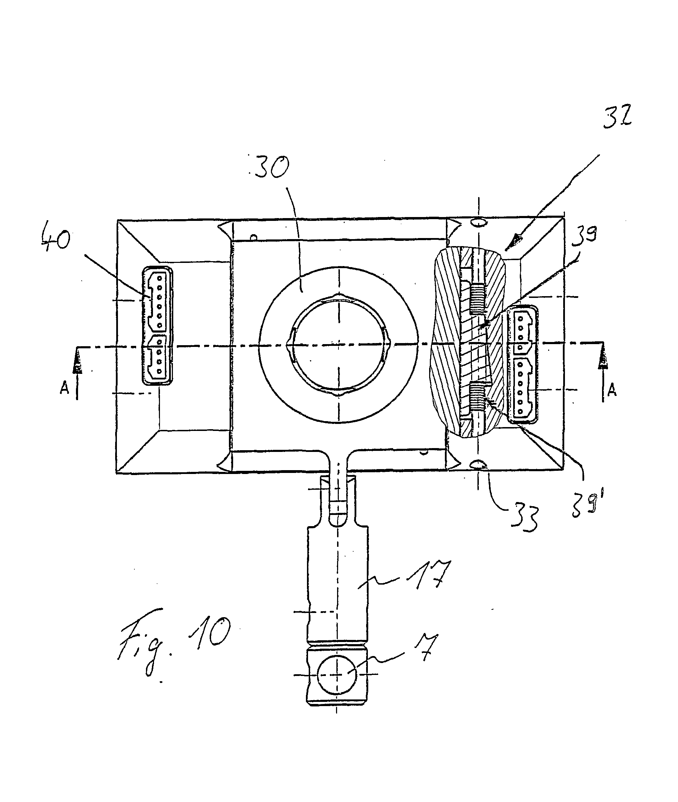

Der Momentensensor ist dabei vorteilhafterweise gemäß der Figur 8 aufgebaut, bei dem eine Pyramidenaufnahme 30 zum Einbringen in den Oberschenkelschaft angeordnet ist. Die Pyramidenaufnahme 30 wird über eine Klemmvorrichtung 32, die in der Figur 10 im Detail dargestellt ist, in einer gewünschten Position auf einem Träger 34 fixiert. Die Pyramidenaufnahme 30 ist, wie durch die Doppelpfeile angedeutet, entlang der Pfeilachse verschieblich, wobei die Pyramidenaufnahme 30 über eine Keilführung in Längsachse unveränderlich gehalten ist. An der Pyramidenaufnahme 30 ist ein Halter 17 für einen Laserpointer 7 angeordnet. Der Laserpointer 7 und der Halter 17 sind ventral angeordnet, wobei der Halter 17 schwenkbar um eine Drehachse in Transversalebene gelagert ist. Ebenfalls ist eine Verdrehung des Laserpointers 7 um eine Achse in Sagittalebene gelagert. Die Klemmvorrichtung 32 ist über Klemmschrauben 39', die durch in Sagittalebene liegende Bohrungen 33 zugänglich sind, lösbar und verriegelbar, so dass die Pyramidenaufnahme 30 auf den Träger 34 verlagerbar fixiert werden kann. Die Pyramidenaufnahme 30, der Träger 34 und das Brückenelement 35 bilden die Anschlussmittel zur Befestigung des nicht dargestellten Oberteiles des Prothesenkniegelenkes mit dem Oberschenkelschaft.

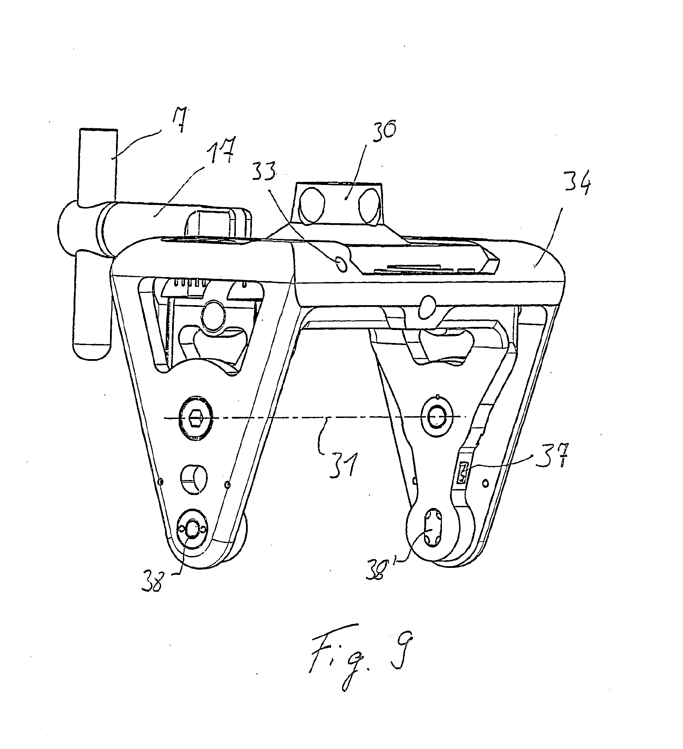

An diesem Träger 34 ist ein Brückenelement 35 festgelegt, das wiederum an dem nicht dargestellten Oberteil des Kniegelenkes befestigt ist. Auf diesem Brückenelement 35 sind Dehnmessstreifen 37, wie sie in der Figur 9 gezeigt sind, aufgebracht, um die auf das Prothesenkniegelenk 3 wirkenden Kräfte und Momente zu ermitteln. Die Dehnmessstreifen 37 sind in der Figur 8 von einer Schutzabdeckung 36 geschützt. Das Brückenelement 35 bildet den Messbalken und ist aus Platzgründen mit den offenen Schenkeln distal ausgerichtet. Um eine möglichst homogene Verformung des Brückenelementes 35 bzw. des Messbalkens zu erreichen, ist dieser gelenkig mit dem Prothesenoberteil verbunden. Dazu sind ein Drehgelenk 38 sowie ein Schiebegelenk 38' an der Verbindung zu dem Prothesenoberteil ausgebildet, wobei das Drehgelenk 38 eine Verdrehung aufgrund von elastischen Verformungen ermöglicht, während das Schiebegelenk 38' einen Längenausgleich zulässt und somit Längsspannungen verhindert. Die Lagerung an dem Oberteil ist unterhalb der Drehachse 31 angeordnet.

Die Figur 9 zeigt in rückwärtiger Ansicht den Momentensensor mit den Dehnmessstreifen 37, die zwischen der Gelenkachse 31 und dem Schiebegelenk 38' bzw. Drehgelenk 38 angeordnet sind.

In der Figur 10 ist in Draufsicht mit einem Teilausschnitt die Klemmvorrichtung 32 gezeigt, die über Klemmschrauben 39' einen Klemmkeil 39 verschieben. Wird die dem Laserpointer 7 zugeordnete Klemmschraube 39' eingedreht, wird über den Klemmkeil 39 eine Kraft in Richtung Frontalebene ausgeübt, wodurch die Pyramidenaufnahme 30, die hier als Verschiebeadapter ausgebildet ist, an dem Träger 34 fixiert wird. Durch eine umgekehrte Drehbewegung der Klemmschraube 39' wird der Klemmkeil 39 in die andere Richtung verfahren und dementsprechend wird die Klemmung gelöst. Die Klemmschraube 39' ist dabei so ausgebildet, dass ein Zugang von beiden Seiten möglich ist. Durch eine entsprechende Lagerung der Klemmschraube 39' ist es möglich, dass sowohl das Öffnen als auch das Schließen der Fixierung bzw. Klemmung von beiden Seiten aus möglich ist.

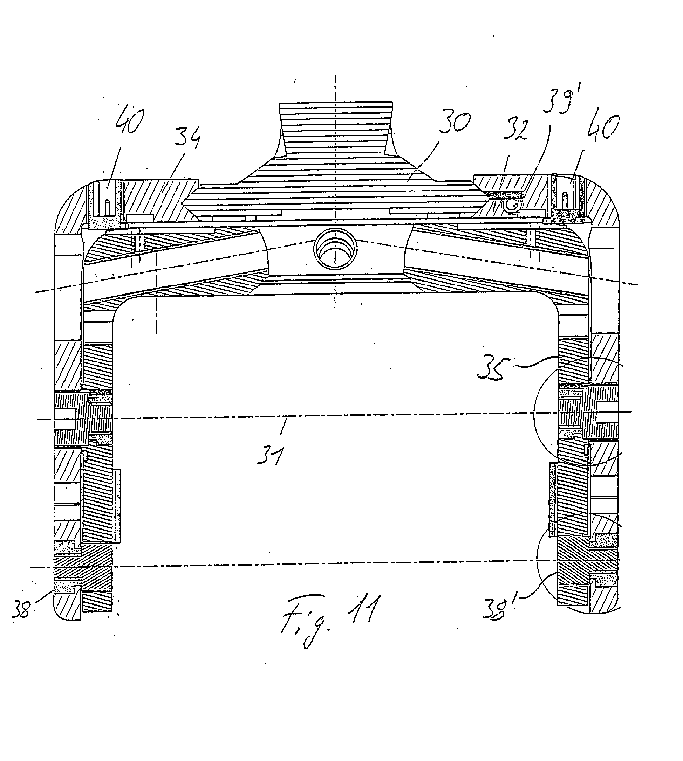

Auf der Oberseite des Halters 34 sind Stecker 40 oder Schnittstellen ausgebildet, die das Auslesen von Sensordaten und eine Übertragung an eine Auswerteeinrichtung, z. B. Computer, ermöglichen. In der Figur 11 ist ein Schnitt A-A dargestellt, der in der Frontalebene ausgeführt ist. Darin ist die Keilführung der Pyramidenaufnahme 30 in dem Träger 34 ebenso zu erkennen, wie die Klemmvorrichtung 32 mit der Klemmschraube 39' und den Schnittstellen 40 zu einem PC.

Die gesamte Vorrichtung zur Erfassung der Kniemomente mit dem Pyramidenadapter 30, dem Träger 34, dem Brückenelement 35 und den Dehnmessstreifen 37 kann nach der erfolgten Evaluierung des Prothesenaufbaus gegen herkömmliche Adapter zur Befestigung des Prothesenkniegelenkes 3 an dem Oberschenkelschaft 2 ausgetauscht sind. Hierzu sind die Maße der Vorrichtung wie der der herkömmlichen Adapter ausgebildet. Es ist auch möglich, die dargestellte Vorrichtung ständig zu nutzen, wobei die Messwerte ständig durch einen Rechner ausgewertet und gegebenenfalls zur Steuerung von Dämpfereinrichtungen ausgewertet werden. Bei einem vorgesehenen Austausch werden die Daten über die Schnittstellen 40 an einem Computer übermittelt, der errechnet, um welche Strecke eine Verlagerung des Pyramidenadapters 3 relativ zu der Gelenkachse 31 erfolgen muss, damit ein stabiler, ein neutraler oder ein dynamischer Aufbau erreicht wird. Das Maß der Versteilung oder die gegenwärtige Einbausituation können auf einem Display ausgegeben werden.

Alternativ zu der dargestellten Ausführungsform können auch andere Kniemomentsensoren vorgesehen und eingesetzt werden.

## Patentansprüche

1. Verfahren zur Überprüfung der Einstellung einer Beinprothese (1), die ein Prothesenkniegelenk (3) aufweist, das einen obenseitigen Anschluss zur Festlegung der Beinprothese (1) an einem Prothesenträger und einen Unterschenkelschaft (4), an dem ein Prothesenfuß (6) befestigt ist, schwenkbar miteinander verbindet, wobei der obenseitige Anschluss relativ zu einer Gelenkachse (31) des Prothesenkniegelenkes (3) verlagerbar ist, **dadurch gekennzeichnet, dass** über Sensoren (10, 11, 37) ein Kniemoment (M_{K}) und eine Axialkraft (F_{AX}) bei einer Prothesennutzung gemessen werden, aus denen ein resultierender Kraftvektor (F_{R}) errechnet wird, und dass der Normalabstand (λ) relativ zu der Gelenkachse (31) und die Lage des resultierenden Kraftvektors zu der Gelenkachse (31) errechnet und überprüft wird, ob und wie weit der resultierende Kraftvektor (F_{R}) in Sagittalebene vor oder hinter der Gelenkachse (31) liegt.

2. Verfahren nach Anspruch 1, **dadurch gekennzeichnet, dass** der resultierende Kraftvektor (F_{R}) mit einem Angriffspunkt an dem obenseitigen Anschluss errechnet wird.

3. Verfahren nach Anspruch 1 oder 2, **dadurch gekennzeichnet, dass** der Normalabstand des resultierenden Kraftvektors (F_{R}) zu der Gelenkachse (31) bestimmt wird.

4. Verfahren nach einem der voranstehenden Ansprüche, **dadurch gekennzeichnet, dass** über einen Knöchelmomentsensor (11) das Knöchelmoment (M_{T}) erfasst wird und dass unter Einbeziehung des Knöchelmomentes (M_{T}), des Kniemomentes (M_{K}), der Axialkraft (F_{AX}) und der Unterschenkelschaftlänge der resultierende Kraftvektor (F_{R}) bestimmt wird.

5. Verfahren nach Anspruch 4, **dadurch gekennzeichnet, dass** der Kraftangriffspunkt des resultierenden Kraftvektors (F_{R}) aus dem Quotienten des Knöchelmomentes (M_{T}) und der Axialkraft (F_{AX}) im Unterschenkelschaft (4) errechnet wird.

6. Verfahren nach einem der voranstehenden Ansprüche, **dadurch gekennzeichnet, dass** das Kniemoment (M_{K}) und die Axialkraft (F_{AX}) während des Gehens gemessen werden.

7. Verfahren nach einem der voranstehenden Ansprüche, **dadurch gekennzeichnet, dass** der resultierende Kraftvektor (F_{R}) unter Ermittlung des aktuellen Kniewinkels (φ) und/oder Oberschenkelwinkels zur Lotlinie ermittelt wird.

8. Verfahren nach einem der voranstehenden Ansprüche, **dadurch gekennzeichnet, dass** der Winkel zwischen dem Unterschenkelschaft (4) und dem Prothesenfuß (6) ermittelt wird und das Kniemoment (M_{K}) und/oder das Knöchelmoment (M_{T}) auf der Grundlage der Erfassung des ermittelten Winkels errechnet wird.

9. Verfahren nach einem der voranstehenden Ansprüche, **dadurch gekennzeichnet, dass** aus den Sensordaten eine Axialkraft (F_{AX}), ein Kniemoment (M_{K}) und ein Knöchelmoment (M_{T}) ermittelt und daraus der Vektor der Bodenreaktionskraft in der Sagittalebene ermittelt wird.

10. Verfahren nach einem der voranstehenden Ansprüche, **dadurch gekennzeichnet, dass** bei einem resultierenden Kraftvektor (F_{R}) hinter der Gelenkachse (31) in der Standphase die Gelenkachse (31) bis hinter den resultierenden Kraftvektor (F_{R}) verschoben wird.

11. Verfahren nach Anspruch 10, **dadurch gekennzeichnet, dass** vor der Verschiebung der Gelenkachse (31) die relative Position des Prothesenfußes (6) zum Unterschenkelschaft (4) markiert und nach der Verschiebung der Gelenkachse (31) die relative Position des Prothesenfußes (6) zum Unterschenkelschaft (4) wieder hergestellt wird.

12. Verfahren nach Anspruch 11, **dadurch gekennzeichnet, dass** die Relativposition des Prothesenfußes (6) festgelegt wird, indem über einen fest an dem Oberschenkelschaft (2) angeordneten Laserpointer (7) ein Laserstrahl auf den Prothesenfuß (6) gerichtet wird, die Auftreffstelle markiert wird und nach der Verlagerung der Gelenkachse (31) die Markierung auf dem Prothesenfuß (6) wieder zur Deckung mit dem Laserstrahl gebracht wird.

13. Verfahren nach einem der Ansprüche 10 bis 12, **dadurch gekennzeichnet, dass** nach der Gelenkachsenverschiebung die Plantarflexionseinstellung des Prothesenfußes (6) korrigiert wird.

14. Verfahren nach Anspruch 13, **dadurch gekennzeichnet, dass** zur Korrektur der Prothesenfußplantarflexionsstellung vor der Gelenkachsenverschiebung von dem Unterschenkelschaft (4) ausgehend eine Referenzlinie (9) am Prothesenfuß (6) und Unterschenkelschaft (4) angezeichnet und nach der Verschiebung der Gelenkachse (31) der Prothesenfuß (6) so ausgerichtet, das die Referenzlinie (9) wieder zur Deckung gebracht wird.

15. Verfahren nach Anspruch 14, **dadurch gekennzeichnet, dass** die Referenzlinie (9) durch eine Justierachse des Prothesenkniegelenkes (3) gelegt wird.

16. Verfahren nach einem der voranstehenden Ansprüche, **dadurch gekennzeichnet, dass** das aktuelle Hüftmoment unter Ermittlung des resultierenden Kraftvektors (F_{R}) und der gegebenen Prothesengeometrie ermittelt wird.

17. Verfahren zur Einstellung einer Beinprothese (1), die ein Prothesenkniegelenk (3) aufweist, das einen obenseitigen Anschluss zur Festlegung der Beinprothese (1) an einem Prothesenträger und einen Unterschenkelschaft (4), an dem ein Prothesenfuß (6) befestigt ist, schwenkbar miteinander verbindet, wobei der obenseitige Anschluss relativ zu einer Gelenkachse (31) des Prothesenkniegelenkes (3) und/oder die Position des Prothesenfußes (6) relativ zu dem Unterschenkelschaft (4) verlagerbar ist, **dadurch gekennzeichnet, dass** über Sensoren (10, 11, 37) ein Kniemoment (M_{K}) und eine Axialkraft (F_{AX}) bei einer Prothesennutzung gemessen werden, aus denen ein resultierender Kraftvektor (F_{R}) errechnet wird, und dass der Normalabstand relativ zu der Gelenkachse (31) und die Lage des resultierenden Kraftvektors (F_{R}) zu der Gelenkachse (31) errechnet und überprüft wird, ob und wie weit der resultierende Kraftvektor (F_{R}) in Sagittalebene vor oder hinter der Gelenkachse (31) liegt und eine Verstellung des obenseitigen Anschlusses relativ zu der Prothesenkniegelenkachse (31) und/oder Streckanschläge (24, 24') für das Prothesenkniegelenk (3) und/oder ein Prothesenfußgelenk in Abhängigkeit von der ermittelten Lage des resultierenden Kraftvektors (F_{R}) vorgenommen wird.

18. Verfahren nach Anspruch 17, **dadurch gekennzeichnet, dass** die Kniegelenksachse (31), die Fußgelenksachse und/oder die Streckanschläge (24, 24') so eingestellt werden, dass der resultierende Kraftvektor (F_{R}) während der Standphase vor der Kniegelenksachse (31) liegt.

19. Vorrichtung zur Messung von Kräften oder Momenten in einer Beinprothese (1) mit einem Prothesengelenk (3), das ein Oberteil zur Verbindung mit einem obenseitigen Anschluss zur Befestigung an einem Prothesenträger und ein mit dem Oberteil gelenkig verbundenes Unterteil aufweist, wobei Anschlussmittel (30, 34, 35) zur Befestigung des Oberteiles an dem obenseitigen Anschluss an dem Oberteil vorgesehen sind, **dadurch gekennzeichnet, dass** Einrichtungen (10, 11, 37) zur Erfassung von Kniekräften oder -momenten an dem Anschlussmittel (30, 34, 35) oder dem Oberteil angebracht sind.

20. Vorrichtung nach Anspruch 19, **dadurch gekennzeichnet, dass** die Anschlussmittel (30, 34, 35) einen in Sagittalebene verschieblich an dem Oberteil gelagerten Adapter (30) aufweisen.

21. Vorrichtung nach Anspruch 19 oder 20, **dadurch gekennzeichnet, dass** der Adapter (30) an einem Halter (34) über eine Klemmeinrichtung (32) festgelegt ist.

22. Vorrichtung nach einem der Ansprüche 19 bis 21, **dadurch gekennzeichnet, dass** zwischen dem Oberteil und dem Adapter (30) ein Träger (34) und/oder Brückenelement (35) angeordnet ist, auf dem oder denen der Adapter (30) verschieblich gelagert ist und das oder die an dem Oberteil festgelegt ist.

23. Vorrichtung nach einem der Ansprüche 19 bis 22, **dadurch gekennzeichnet, dass** an dem Brockenelement (35) Dehnungsmessstreifen (37) zur Ermittlung der wirksamen Kniekräfte und -momente angebracht sind.

24. Vorrichtung nach einem der Ansprüche 19 bis 23, **dadurch gekennzeichnet, dass** der Adapter (30) oder der Adapter (30) mit dem Träger (34) und/oder dem Brückenelement (35) eine Einbauhöhe aufweist, die der Einbauhöhe der Anschlussmittel entspricht, die nach der Einstellung des Prothesenaufbaues eingesetzt werden.

25. Vorrichtung nach einem der Ansprüche 19 bis 24, **dadurch gekennzeichnet, dass** an den Anschlussmitteln (34, 35) ein Träger (17) für eine Markierungseinrichtung, insbesondere Laserpointer (7) befestigt ist.

26. Vorrichtung nach einem der Ansprüche 19 bis 25, **dadurch gekennzeichnet, dass** der obenseitige Anschluss als Oberschenkelschaft (2) ausgebildet ist.

## Claims

1. Method for verifying the adjustment of a leg prosthesis (1) which has a prosthetic knee joint (3), which pivotably interconnects an upper connection, for fixing the leg prosthesis (1) on a wearer of the prosthesis, and a lower leg shaft (4), to which a prosthetic foot (6) is fastened, the upper connection being displaceable in relation to a joint axis (31) of the prosthetic knee joint (3), **characterized in that** a knee moment (M_{K}) and an axial force (F_{AX}) are measured by means of sensors (10, 11, 37) when a prosthesis is being used and are used to calculate a resultant force vector (F_{R}) and **in that** the normal distance (λ) relative to the joint axis (31) and the position of the resultant force vector in relation to the joint axis (31) is calculated and it is verified whether and to what extent the resultant force vector (F_{R}) lies in front of or behind the joint axis (31) in the sagittal plane.

2. Method as claimed in claim 1, **characterized in that** the resultant force vector (F_{R}) with a point of attack on the upper connection is calculated.

3. Method as claimed in claim 1 or 2, **characterized in that** the normal distance of the resultant force vector (F_{R}) from the joint axis (31) is determined.

4. Method as claimed in one of the preceding claims, **characterized in that** the ankle moment (M_{T}) is detected by means of an ankle moment sensor (11) and **in that**, with inclusion of the ankle moment (M_{T}), the knee moment (M_{K}), the axial force (F_{AX}) and the length of the lower leg shaft, the resultant force vector (F_{R}) is determined.

5. Method as claimed in claim 4, **characterized in that** the point of force attack of the resultant force vector (F_{R}) is calculated from the quotient of the ankle moment (M_{T}) and the axial force (F_{AX}) in the lower leg shaft (4).

6. Method as claimed in one of the preceding claims, **characterized in that** the knee moment (M_{K}) and the axial force (F_{AX}) are measured during walking.

7. Method as claimed in one of the preceding claims, **characterized in that** the resultant force vector (F_{R}) is determined by determining the current knee angle (Φ) and/or the upper leg angle in relation to the vertical line.

8. Method as claimed in one of the preceding claims, **characterized in that** the angle between the lower leg shaft (4) and the prosthetic foot (6) is determined and the knee moment (M_{K}) and/or the ankle moment (M_{T}) is calculated on the basis of the detection of the angle determined.

9. Method as claimed in one of the preceding claims, **characterized in that** the sensor data are used to determine an axial force (F_{AX}), a knee moment (M_{K}) and an ankle moment (M_{T}), from which the vector of the ground reaction force in the sagittal plane is determined.

10. Method as claimed in one of the preceding claims, **characterized in that**, in the case of a resultant force vector (F_{R}) behind the joint axis (31) in the standing phase, the joint axis is displaced to behind the resultant force vector (F_{R}).

11. Method as claimed in claim 10, **characterized in that** the relative position of the prosthetic foot (6) with respect to the lower leg shaft (4) is marked before the displacement of the joint axis (31) and the relative position of the prosthetic foot (6) with respect to the lower leg shaft (4) is restored after the displacement of the joint axis (31).

12. Method as claimed in claim 11, **characterized in that** the relative position of the prosthetic foot (6) is fixed by a laser beam being directed onto the prosthetic foot (6) by means of a laser pointer (7) fixedly arranged on the upper leg shaft (2), the point of impingement marked and the marking on the prosthetic foot (6) made to coincide again with the laser beam after the displacement of the joint axis (31).

13. Method as claimed in one of claims 10 to 12, **characterized in that**, after the displacement of the joint axis, the plantar flexion adjustment of the prosthetic foot (6) is corrected.

14. Method as claimed in claim 13, **characterized in that**, to correct the position of the plantar flexion of the prosthetic foot, before the displacement of the joint axis, a reference line (9) is drawn from the lower leg shaft (4) on the prosthetic foot (6) and the lower leg shaft (4) and, after the displacement of the joint axis (31), the prosthetic foot (6) is aligned in such a way that the reference line (9) is made to coincide again.

15. Method as claimed in claim 14, **characterized in that** the reference line (9) is placed through an adjusting axis (3) of the prosthetic knee joint.

16. Method as claimed in one of the preceding claims, **characterized in that** the current hip moment is determined by determining the resultant force vector (F_{R}) and the given geometry of the prosthesis.

17. Method for adjusting a leg prosthesis (1) which has a prosthetic knee joint (3), which pivotably interconnects an upper connection for fixing the leg prosthesis on a wearer of the prosthesis, and a lower leg shaft (4), to which a prosthetic foot (6) is fastened, the upper connection being displaceable in relation to a joint axis (31) of the prosthetic knee joint (3) and/or the position of the prosthetic foot (6) in relation to the lower leg shaft (4), **characterized in that** a knee moment (M_{K}) and an axial force (F_{AX}) are measured by means of sensors (10, 11, 37) when a prosthesis is being used, the knee moment and the axial force being used to calculate a resultant force vector (F_{R}), and **in that** the normal distance in relation to the joint axis (31) and the position of the resultant force vector (F_{R}) in relation to the joint axis (31) are calculated and it is verified whether and to what extent the resultant force vector (F_{R}) lies in front of or behind the joint axis (31) in the sagittal plane, and an adjustment of the upper connection in relation to the axis (31) of the prosthetic knee joint and/or extension stops (24, 24') for the prosthetic knee joint (3) and/or a prosthetic foot joint is performed in dependence on the determined position of the resultant force vector (F_{R}).

18. Method as claimed in claim 17, **characterized in that** the knee joint axis (31), the foot joint axis and/or the extension stops (24, 24') are adjusted in such a way that the resultant force vector (F_{R}) lies in front of the knee joint axis (31) during the standing phase.

19. Apparatus for the measurement of forces or moments in a leg prosthesis (1) with a prosthetic joint (3), which has an upper part for the connection to an upper connection for fastening on a wearer of a prosthesis, and a lower part jointedly connected to the upper part, connecting means (30, 34, 35) being provided on the upper part for fastening the upper part to the upper connection, **characterized in that** devices (10, 11, 37) for detecting knee forces or moments are provided on the connecting means (30, 34, 35) or the upper part.

20. Apparatus as claimed in claim 19, **characterized in that** the connecting means (30, 34, 35) have an adapter (30) mounted displaceably in the sagittal plane on the upper part.

21. Apparatus as claimed in claim 19 or 20, **characterized in that** the adapter (30) is fixed on a holder (34) by means of a clamping device (32).

22. Apparatus as claimed in one of claims 19 to 21, **characterized in that** arranged between the upper part and the adapter (30) is a carrier (34) and/or bridge element (35), on which the adapter (30) is displaceably mounted and which is or are fixed to the upper part.

23. Apparatus as claimed in one of claims 19 to 22, **characterized in that** strain gauges (37) for determining the effective knee forces and moments are provided on the bridge element (35).

24. Apparatus as claimed in one of claims 19 to 23, **characterized in that** the adapter (30) or the adapter (30) with the carrier (34) and/or the bridge element (35) has a fitting height that corresponds to the fitting height of the connecting means that are used after the adjustment of the prosthesis setup.

25. Apparatus as claimed in one of claims 19 to 24, **characterized in that** a carrier (17) for a marking device, in particular a laser pointer (7), is fastened to the connecting means (34,35).

26. Apparatus as claimed in one of claims 19 to 25, **characterized in that** the upper connection is formed as an upper leg socket (2).

## Revendications

1. Procédé pour le contrôle du réglage d'une prothèse de jambe (1) qui comporte une articulation de genou de prothèse (3) qui relie l'un à l'autre avec faculté de pivotement un raccord supérieur pour immobiliser la prothèse de jambe (1) à un porteur de prothèse et une tige de jambe inférieure (4) à laquelle est fixé un pied de prothèse (6), dans lequel le raccord supérieur est déplaçable par rapport à un axe d'articulation (31) de l'articulation de genou de prothèse (3),
**caractérisé en ce que** l'on mesure au moyen de capteurs (10, 11, 37) un couple de genou (M_{K}) et une force axiale (F_{AX}) lors d'une utilisation de la prothèse, à partir desquels on calcule un vecteur de force résultant (F_{R}), et **en ce que** l'on calcule la distance normale (1) par rapport à l'axe d'articulation (31) et la position du vecteur de force résultant par rapport à l'axe d'articulation (31) et on contrôle si le vecteur de force résultant (F_{R}) dans le plan sagittal se trouve devant ou derrière l'axe d'articulation (31) et à quelle distance.

2. Procédé selon la revendication 1, **caractérisé en ce que** le vecteur de force résultant (F_{R}) est calculé avec un point d'attaque au niveau du raccord supérieur.

3. Procédé selon la revendication 1 ou 2, **caractérisé en ce que** la distance normale du vecteur de force résultant (F_{R}) est déterminée par rapport à l'axe d'articulation (31).

4. Procédé selon l'une des revendications précédentes, **caractérisé en ce que** l'on détecte le couple de cheville (M_{T}) au moyen d'un capteur de couple de cheville (11), et **en ce que** l'on détermine le vecteur de force résultant (F_{R}) en tenant compte du couple de cheville (M_{T}), du couple de genou (M_{K}), de la force axiale (F_{AX}), et de la longueur de la jambe inférieure.

5. Procédé selon la revendication 4, **caractérisé en ce que** le point d'attaque du vecteur de force résultant (F_{R}) est calculé à partir du quotient du couple de cheville (M_{T}) et de la force axiale (F_{AX}) dans la tige de jambe inférieure (4).

6. Procédé selon l'une des revendications précédentes, **caractérisé en ce que** le couple de genou (M_{K}) et la force axiale (F_{AX}) sont mesurés pendant la marche.

7. Procédé selon l'une des revendications précédentes, **caractérisé en ce que** le vecteur de force résultant (F_{R}) est déterminé en déterminant l'angle actuel du genou (Φ) et/ou l'angle de la cuisse par rapport à une ligne verticale.

8. Procédé selon l'une des revendications précédentes, **caractérisé en ce que** l'on détermine l'angle entre la tige de jambe inférieure (4) et le pied de prothèse (6), et l'on calcule le couple de genou (M_{K}) et/ou le couple de cheville (M_{T}) en se basant sur la détection de l'angle déterminé.

9. Procédé selon l'une des revendications précédentes, **caractérisé en ce que** l'on détermine à partir des données de capteur une force axiale (F_{AX}), un couple de genou (M_{K}) et un couple de cheville (M_{T}), et **en ce que** l'on détermine à partir de ceux-ci le vecteur de la force de réaction au sol dans le plan sagittal.

10. Procédé selon l'une des revendications précédentes, **caractérisé en ce que** lorsque le vecteur de force résultant (F_{R}) est derrière l'axe d'articulation (31) dans la phase stationnaire debout, l'axe d'articulation (31) est déplacé jusqu'en arrière du vecteur de force résultant (F_{R}).

11. Procédé selon la revendication 10, **caractérisé en ce qu'**avant le déplacement de l'axe d'articulation (31) on marque la position relative du pied de prothèse (6) par rapport à la tige de jambe inférieure (4), et **en ce que**, après déplacement de l'axe d'articulation (31), on rétablit la position relative du pied de prothèse (6) par rapport à la tige de jambe inférieure (4).

12. Procédé selon la revendication 11, **caractérisé en ce que** la position relative du pied de prothèse (6) est fixée, en orientant, au moyen d'un pointeur à laser (7) agencé sur la tige de la cuisse (2), un rayon laser vers le pied de prothèse (6), on marque l'emplacement d'incidence et, après déplacement de l'axe d'articulation (31), on amène le marquage sur le pied de prothèse (6) à nouveau en coïncidence avec le rayon laser.

13. Procédé selon l'une des revendications 10 à 12, **caractérisé en ce que**, après le déplacement de l'axe d'articulation, le réglage de flexion plantaire du pied de prothèse (6) est corrigé.

14. Procédé selon la revendication 13, **caractérisé en ce que**, pour la correction de la position de flexion plantaire du pied de prothèse et avant le déplacement de l'axe d'articulation en partant de la tige de jambe inférieure (4), on dessine une ligne de référence (9) sur le pied de prothèse (6) et sur la tige de jambe inférieure (4) et, après le déplacement de l'axe d'articulation (31) on oriente le pied de prothèse (6) de telle façon que la ligne de référence (9) est à nouveau amenée en coïncidence.

15. Procédé selon la revendication 14, **caractérisé en ce que** la ligne de référence (9) est placée à travers un axe d'ajustement de l'articulation de genou de prothèse (3).

16. Procédé selon l'une des revendications précédentes, **caractérisé en ce que** le couple de hanche actuel est déterminé en déterminant le vecteur de force résultant (F_{R}) et la géométrie de prothèse donnée.

17. Procédé pour le réglage d'une prothèse de jambe (1) qui comporte une articulation de genou de prothèse (3) qui relie l'un à l'autre avec faculté de pivotement un raccord supérieur pour immobiliser la prothèse de jambe (1) à un porteur de prothèse et une tige de jambe inférieure (4) à laquelle est fixé un pied de prothèse (6), dans lequel le raccord supérieur est déplaçable par rapport à un axe d'articulation (31) de l'articulation de genou de prothèse (3) et/ou la position du pied de prothèse (6) est déplaçable par rapport à la tige de jambe inférieure (4), **caractérisé en ce que** l'on mesure au moyen de capteurs (10, 11, 37) un couple de genou (M_{K}) et une force axiale (F_{AX}) lors d'une utilisation de la prothèse, à partir desquels on calcule un vecteur de force résultant (F_{R}), et **en ce que** l'on calcule la distance normale (1) par rapport à l'axe d'articulation (31) et la position du vecteur de force résultant par rapport à l'axe d'articulation (31) et on contrôle si le vecteur de force résultant (F_{R}) dans le plan sagittal se trouve devant ou derrière l'axe d'articulation (31) et à quelle distance, et on effectue un déplacement du raccord supérieur par rapport à l'axe d'articulation de genou de prothèse (31) et/ou par rapport à des butées d'extension (24, 24') pour l'articulation de genou de prothèse (3) et/ou une articulation de pied de prothèse en fonction de la position déterminée du vecteur de force résultant (F_{R}).

18. Procédé selon la revendication 17, **caractérisé en ce que** l'axe d'articulation de genou (31), l'axe d'articulation de pied, et/ou les butées d'extension (24, 24') sont réglés de telle façon que le vecteur de force résultant (FR) pendant la phase de station debout, se trouve devant l'axe d'articulation de genou (31).

19. Dispositif pour la mesure de force ou de couple dans une prothèse de jambe (1) avec une articulation de prothèse (3) qui comprend une partie supérieure destinée à la liaison avec un raccord supérieur pour la fixation sur un porteur de prothèse et une partie inférieure reliée de manière articulée à la partie supérieure, dans lequel il est prévu des moyens de raccordement (30, 34, 35) pour la fixation de la partie supérieure sur le raccord supérieur de la partie supérieure, **caractérisé en ce que** des moyens (10, 11, 37) pour la détection de forces ou de couples de genou sont montés sur les moyens de raccordement (30, 34, 35) ou sur la partie supérieure.

20. Dispositif selon la revendication 19, **caractérisé en ce que** les moyens de raccordement (30, 34, 35) comprennent un adaptateur (30) monté sur la partie supérieure et déplaçable dans le plan sagittal.

21. Dispositif selon la revendication 19 ou 20, **caractérisé en ce que** l'adaptateur (30) est immobilisé sur une monture (34) via un moyen de serrage (32).

22. Dispositif selon l'une des revendications 19 à 21, **caractérisé en ce qu'**un support (34) et/ou un élément en pontet (35) est agencé entre la partie supérieure et l'adaptateur (30), support ou élément sur lequel l'adaptateur (30) est monté déplaçable, et qui est immobilisé sur la partie supérieure.

23. Dispositif selon l'une des revendications 19 à 22, **caractérisé en ce que** des jauges de contraintes (37) sont montées sur l'élément en pontet (35) pour la détermination des forces et des couples agissant au niveau du genou.

24. Dispositif selon l'une des revendications 19 à 23, **caractérisé en ce que** l'adaptateur (30) ou encore l'adaptateur (30) avec le support (34) et/ou avec l'élément en pontet (35) présente une hauteur structurelle qui correspond à la hauteur structurelle des moyens de raccordement qui sont employés après réglage de la structure de prothèse.

25. Dispositif selon l'une des revendications 19 à 24, **caractérisé en ce qu'**un support (17) pour un dispositif de marquage, en particulier pour un pointeur laser (7), est fixé sur les moyens de raccordement (34, 35).

26. Dispositif selon l'une des revendications 19 à 25, **caractérisé en ce que** le raccord supérieur est réalisé sous forme de tige de cuisse (2).
